# EUROPEAN PATENT SPECIFICATION

(11) **EP 1 696 937 B1**
(45) Date of publication and mention of the grant of the patent: **11.03.2009**
(21) Application number: 04808876.9
(22) Date of filing: 10.12.2004
(51) Int. Cl.: A61K 31/733, A61K 31/718, A23L 1/308, A61P 1/00, A61P 3/14

(54) **PREBIOTIC COMBINATION PRODUCTS**
PRÄBIOTISCHE KOMBINATIONSPRODUKTE
PRODUITS DE COMBINAISON PREBIOTIQUES

(30) Priority: 12.12.2003 NO 20035566
(43) Date of publication of application: 06.09.2006
(73) Proprietor: Nova Biotics AS, N-1387 Asker (NO)
(72) Inventor: PIENE, Jan, Yngvar, N-1387 Asker (NO)
(74) Representative: Sønstevold, Anne
(86) International application number: PCT/NO2004/000382
(87) International publication number: WO 2005/056023

(56) References cited:
- EP-A1- 0 756 828
- EP-A1- 1 175 905
- EP-A1- 1 243 273
- EP-A1- 1 386 546
- WO-A1-01/64225
- US-A1- 2002 172 724
- US-A1- 2003 049 313
- US-B1- 6 203 797
- YOUNES H. ET AL: 'Effects of two fermentable carbohydrates (inulin and resistant starch) and their combination on calcium and magnesium balance in rats' BRITISH JOURNAL OF NUTRITION vol. 86, 2001, pages 479 - 485, XP002986200
- MOREAU N.M. ET AL: 'Restoration of the integrity of rat caeco-colonic mucosa by resistant starch, but not by fructo-oligosaccharides, in dextran sulfate sodium-induced experimental colitis' BRITISH JOURNAL OF NUTRITION vol. 90, 2003, pages 75 - 85, XP002987301
- ANDOH A. ET AL: 'Role of Dietary Fiber and Short-Chain Fatty Acids in the Colon' CURRENT PHARMACEUTICAL DESIGN vol. 9, 2003, pages 347 - 358, XP002987302

## Description

The present invention relates to a chewable tablet comprising a prebiotic combination product of inulin and/or oligofructose and resistant starch, and wherein said combination product has been subjected to direct mixing and compression or a wet granulation process and drying before tableting.

The invention describes prebiotic fiber combination products containing inulin/oligofructose and resistant starch and methods for making these with a high acceptability and consumer acceptance for the prophylaxis and treatment of inflammatory bowel disease (IBD), calcium and magnesium deficiency including osteoporosis, food intolerances, the general regulation of the digestion, stimulation of the immune system, prophylaxis of colon cancer, energy management and protection of the gut microbial ecosystem in man. In particular it describes methods for making chewable tablets as the preferred dosage form for the prebiotic supplement. The chewable tablet formulations in the invention have improved characteristics with respect to sensoric properties, tablet compression, friability and stability.

Recommended dietary intake of fiber for adults generally fall in the range of 20 to 35 g/day. However the average consumption of fiber is far less than this level and frequently in the range of 10 to 15 g/day. There is thus a general need to make attractive products to be ingested orally in order to increase the availability and amount of fiber to be ingested by the public.

Traditionally fiber has been administered as powders to be reconstituted by the addition of water. These have usually been highly unattractive products due to a bland and fibrous taste and because of the swelling of the soluble fiber components in the products giving rise to a slimy consistency. Likewise the attempt to formulate fiber components into tablets have been largely unsuccessful due to the large bulk mass of the fiber to be administered giving rise to an unrealistically high number of tablets to be taken by the consumer.

US 6,750,331 B1: Oligosaccharide-supplying compositions, describes the difficulties relating to the oral administration of oligosaccharides where existing formulations are said to be suffering from poor sensoric properties and inferior stability. Chewable tablets containing oligosaccharides are said to have a drawback of sticking to the teeth (toothpacking) and a poor stability due to the amorphous nature of oligosaccharides which impart a high degree of hygroscopicity and lump formation. The cited document tempt to solve this problem by making a chewable tablet containing up to 80% of oligosaccharide and with the inclusion of effervescent components.

US 6,455,068 B1: Dietary fiber delivery system, describes a chewable tablet containing at least 50% of inulin or oligofructose by weight of the tablet. The method of manufacture of the tablets was by direct mixing and subsequent compression in a tablet machine to make tablets with a preferred tablet weight in the range of 3 to 5 grams. The chewable tablet consisted of 30 to 60% of the low viscosity fiber component, the other component mainly being sorbitol which was added in order to aid the chewable consistency of the product.

In principle when formulating tablets there is a general aim to reduce the need of tablet excipients to a minimum in order to keep the tablet size down and in order to reduce cost. However it is of vital importance to produce a robust tablet formulation which compress well during the tableting step without any evidence of capping or scale-off. This can be documented by a linear tablet compression curve over a wide range of tableting pressures and with the production of tablets with a very small friability percentage at normal operating tableting pressures and tableting speeds.

There is thus a need to produce improved dosage forms containing inulin and oligofructose with improved characteristics related to sensoric properties, a reduced susceptibility to moisture and with satisfactory tablet characteristics like ease of compression and low friability. This should preferably be achieved without the need of extra excipients.
The described invention concerns the formulation of a prebiotic combination product consisting of a non-gelling and soluble component being inulin or oligofructose in combination with an insoluble fiber component being resistant starch, optionally containing another insoluble fiber component not being a prebiotic and optionally containing a soluble filler component.
The present invention discerns itself from US 6,455,068 in that the total fiber content per tablet can be as high as 98%, the content of inulin or oligofructose being less than 50% in the tablet and that the preferred tablet weight being less than 3 grams. Contrary to US 6,455,068 it has been found that a tablet consisting of inulin alone does not compress well and has an inferior and unsatisfactory friability. It has also been seen that a chewable tablet based on inulin or oligofructose alone gives rise to a product with unpleasant sensoric properties due to toothpacking.
Surprisingly it has been found that a combination of inulin/oligofructose and resistant starch has greatly improved tablet compressibility and friability. This effect has been seen with tablets based on direct compression and also where the tablet is based on a fluid bed granulate. The formulation may have a very high fiber content of up to 98% without the need of compression aid excipients, tablet binders and soluble filler aid materials. Furthermore it has been found out that the combination of enriched inulin and resistant starch gives rise to a chewable tablet with improved sensoric properties compared to a tablet based on inulin/oligofructose alone. The inclusion of resistant starch also makes the tablet more resistant towards moisture which may be a problem during the production of the tablet but which also present a stability problem in a consumer situation with resultant inferior sensoric properties.

Food intolerances and chronic bowel diseases like ulcerative colitis, Crohn's disease and irritable bowel syndrome are difficult diseases to treat. There is no present cure for the diseases and the patient can only get ameliorative effect from various drug regimes. In cases of ulcerative colitis it may be necessary to give corticosteroids in order to control the inflammation which in turn after long term usage can results in serious and chronic side-effects.

There is thus a need to find effective dosage forms and products which work in a prophylactic way without causing any serious side-effects after long term usage. The exact cause of ulcerative colitis is not known, but there are various theories to its cause.

A review article in Lancet (vol.359, Jan.26, 2002) mentions the influence of the enteric micro flora in the colon in addition to genetic and immunologic factors as possible causes to the disease. It is suggested that ulcerative colitis results from environmental factors triggering a breakdown in the regulatory constraints on mucosal immune responses to enteric bacteria in genetically susceptible individuals. Probiotics is further suggested as a possible therapy in order to manipulate the normal intestinal flora towards a more beneficial composition which does not produce harmful autoantigens. Probiotics is here defined as living bacteria which has a beneficial and health-promoting effect in the gastrointestinal system. Lactobacillus and bifidobacterium species, which do not have pro-inflammatory effect, have been selected for probiotic therapy. Another review article in the Lancet (vol.359, Joan.5, 2002) suggests that intestinal inflammation in Crohn's disease arises from abnormal immune reactivity to bacterial flora in the intestine of individuals who are genetically susceptible.
Likewise a report in Lancet (vol.352, Oct. 1998) suggests that irritable bowel syndrome may be caused by abnormal colonic fermentation.

Another method of influencing the enteric microflora is by giving prebiotics which stimulate the growth and multiplication of beneficial microorganisms like lactobacillus and bifidobacterium species in the colon.

Prebiotics is here defined as non-digestible but fermentable carbohydrates which in a beneficial fashion influence the organism by stimulating the growth or activity of one or a limited number of microorganisms which are already present in the colon and which results in an improvement of the health of the host.
Examples of prebiotica are non-digestible carbohydrates and fibre like short chain fructooligosaccharide (_{sc}FOS), oligofructose, inulin and resistant starch. It is increasing evidence that these special types or sub-classes of fermentable non-digestible polysaccharides can have a modulating effect on the immune system and on the microflora in the colon. The increased fermentation from beneficial bacteria like lactobacillus and bifidobacterium will result in a an increased number of these beneficial bacteria and a decrease of the pH which will both prevent more harmful bacteria like E.coli and Salmonella to take residence in the colon. The prebiotic fibres will also cause an increased production of butyric acid which acts as the main source of energy to the colonocytes lining the epithelium of the colon. A sufficient supply of butyric acid is thought to be important for the maintenance of a healthy epithelium in the colon thus preventing inflammatory disease and also thought to be important for the prophylaxis of colon cancer.

The prebiotica also exert an enhancement of the bioavailability of important macrominerals like calcium and magnesium. An optimum absorption of calcium is important throughout life especially in the adolescent population which need to achieve a satisfactory peak bone mass and in the aging population who are subjected to a negative calcium balance with a resultant development of osteoporosis.
Abrahams S., Griffin I (2000) carried out a study on 29 adolescent girls which confirmed an 18 % increased calcium absorption upon digestion of 8 grams of enriched inulin (Raftilose Synergy 1).

A recent study by Dr. Anne Friedlander et al. (2003) on 15 post-menopausal women showed that 6 grams of Raftilose Synergy 1 increased the absorption of calcium and magnesium by 20% when compared to a placebo treatment.

Resistant starch and "the butyrate revolution" by Fred Brouns (Trends in Food Science & Technology 13, 2002) gives an overview of the present knowledge concerning resistant starch and the physiological effect of butyrate. It states that in vitro studies as well as animal models indicate that butyrate have the potential to support the maintenance of a healthy gut and to reduce risk factors that are involved in the development of gut inflammation as well as colorectal cancer. Resistant starch induces a high butyrate production and that this production is also present in the descending colon and rectum where the risk of developing cancer is a 1000 fold higher than in other parts of the intestine. Butyrate functions as an energy supply to the colonocytes in the epithelial lining of the gut which in turn maintain a healthy gut wall function.

The overview also states that some experimental studies suggest that intakes in the order of ≥ 20 g/day of resistant starch may be needed in order to obtain some of the bowel related benefits.
An acceptance study is further described where dosages higher than 20 g/day were investigated with respect to digestive tolerance.

Added-extra Ingredients by Thomas Draguhn (Food Processing July 2002) gives an overview of the potential health benefits of resistant starch and especially Actistar based on retrograded amylose starch. The present consumption of resistant starch is 3-7 g in developed countries despite the fact that it is recommended that around 20 g per day are required to obtain some of its bowel-related benefits.
Roberfroid et al. (1998) concluded from their compilation work that in a large population there seems to be no dose-response effect of "non digestible oligosaccharides" with respect to the increase in numbers of bifidogenic bacteria for daily intakes between 4 and 40 g/d. This is because the bifidogenic effect is inversely related to the volunteers initial bifidus count.

Raw potato starch and short-chain fructo-oligosaccharides affect the composition and metabolic activity of rat intestinal microbiota differently depending on the caecocolonic segment involved by G.M. Le Bay et al. (Journal of Applied Microbiology 94, 2003). This article states that fructo-oligosaccharides (FOS) and resistant potato starch (RPS) were demonstrated to have different fermentation kinetics where RPS was more slowly metabolised throughout the colon whereas FOS was mainly metabolised in the caecum and proximal colon. Therefore, the combined ingestion of both FOS and RPS seems advisable in view of the health-promoting effect throughout the colon.

From prior art it can be concluded that the following information was already known at the time when the invention was made:
Inulin and oligofructose based on chicory is a prebiotic which causes a selective fermentation by the bifidobacteria in the first and middle part of the colon. Here the beneficial bacteria produce short chain fatty acids and especially butyric acid which is thought to be important for the functioning of a healthy epithelium in the colon. Similarly resistant starch is known to be a prebiotic and the most powerful substrate for beneficial bacteria generating butyric acid in the entire length of the colon and especially in the last part of the colon.

Thus present knowledge suggests that a prebiotic combination of inulin/oligofructose and resistant starch may be beneficial for colonic health as opposed to any of the prebiotics taken alone.

There are no references concerning any clinical studies on patients with inflammatory bowel diseases or on patients with food intolerances. Thus any dosage for the prophylactic or therapeutic use of these prebiotics in the referred diseases is thus unknown.

Thus it is not known whether a particular prebiotic component or a combination of prebiotics would be beneficial in the stated diseases or what dosage levels which may exert a prophylactic or therapeutic effect.

The presents invention relates to a prebiotic combination product, comprising inulin and/or oligofructose and resistant starch as functional ingredients, optionally a soluble filler and optionally an insoluble fiber component not being a prebiotic; and wherein said combination product has been subjected to direct mixing and compression or a wet granulation process and drying before tableting, and said dried prebiotic product being in the form of chewable tablets as the preferred dosage form.

The present invention discerns itself from the present knowledge with respect to the following new information:
- Contrary to the present knowledge it has been found that surprisingly low dosages of inulin/oligofructose and resistant starch in combination have clinical effect for patients with ulcerative colitis and for patients with food intolerances.
- A common problem with prebiotics is that they increase intestinal discomfort due to an increased incidence of flatulence and bloatedness. Due to the small dosages of the prebiotic supplement this does not occur. Again this will increase the patient compliance and the acceptability of the supplement in long term usage.
- A small dosage of the two prebiotics facilitates the formulation of effective and compact dosage forms with increased patient compliance.
- A preferred dosage form is chewable tablets with the following new characteristics:
   ○ a beneficial effect is seen between inulin or enriched inulin and resistant starch with respect to tablet compression and friability
   ○ very high loading of fiber components per tablet, this being as high as 98% and without the need of any extra tablet excipients
   ○ very good sensoric properties despite the very high fiber load per tablet
   ○ improved stability and resistance towards humidity
- Finally will a supplement containing only small amounts of the active prebiotic components constitute a cost effective dosage form for the consumer. This is especially the case in the treatment of inflammatory bowel disease where the patient is dependent on expensive drug regimens.
- Treating inflammatory bowel disease, food intolerances and digestive problems with natural fibre components does not cause any serious side-effects as opposed to drug regimens containing drugs like cortisone.

The reported necessary dosage of inulin/oligofructose is 5 grams in order for the prebiotic to exert a gastrointestinal bifidogenic effect. The minimum dosage of resistant starch necessary to exert a bifidogenic effect is not known. However the manufacturer of Actistar 11700 recommends a dosage of 5 grams per serving.

An acceptance study on 18 volunteers and a long term study on two patients with ulcerative colitis and one person with food intolerance have been carried out with the prebiotic food supplement described in example 1.

The two studies are described in example 2 where both an excellent acceptability and long term effect for the two patients with ulcerative colitis and for the one patient with food intolerance are shown.

The results of the acceptance study over 5 weeks are depicted in the figures where:
Fig. 1 is a graph which shows the crushing strength and friability as a function of tableting pressure for two prebiotic fiber tablets based on direct compression.
Fig. 2 is a graph which shows the crushing strength and friability as a function of tableting pressure for two prebiotic fiber tablets based on a fluid bed granulate and direct compression.
Fig. 3 is a graph which shows number of bowel movements to pass faeces as a function of time.
Fig. 4 is a graph which shows bloatedness as a function of time.
Fig. 5 is a graph which shows intestinal pressure as a function of time.
Fig. 6 is a graph which shows intestinal sounds as a function of time.
Fig. 7 is a graph which shows consistency of faeces as a function of time.

The amounts of inulin/oligofructose and resistant starch (Actistar 11700) in the prebiotic supplement are both 0.7 grams. This is 7 times less than the quoted effective or recommended dosages for inulin/oligofructose and resistant starch respectively. This combination of small dosages of the prebiotic active ingredients facilitates effective and compact dosage forms like chewable tablets and sachet products.

Dietary Reference Intakes for Energy, Carbohydrates, Fibre, Fat, Protein and Amino Acids 2002 by the Institute of Medicine in the USA defines functional fibre as isolated, nondigestible carbohydrates that have beneficial physiological effects in humans. Functional fibre consists of isolated or extracted nondigestible carbohydrates where the isolation or extraction process has been carried out by chemical, enzymatic or aqueous steps. Synthetically manufactured or naturally occurring isolated oligosaccharides and manufactured resistant starch are included in this definition.
Since both oligosaccharides and resistant starch is included in the composition of the invention a more thorough definition of the two functional fibres are defined below.

Inulin and oligofructose are naturally occurring in a variety of plants such as wheat, chicory, onions and Jerusalem artichoke. Inulin is a polydisperse β-(2,1)-linked fructan with a glucose molecule at the end of each fructose chain. The chain length is usually 2 to 60 units with an average degree of polymerisation of 10. The β-(2,1) linkage is resistant to enzymatic digestion. Oligofructose may be formed by partial hydrolysis of inulin with an average degree of polymerisation of between 2 to 10.
Short chain fructooligosaccharide (_{sc}FOS) is a defined mixture of glucose-terminated fructose chains with a maximum chain length of 5 units. It is derived from sugar cane by an enzymatic process.

Inulin and oligofructose are available from Orafti SA, Tienen, Belgium under the trade names Raftiline and Raftilose respectively. Short chain fructooligosaccharide is available under the trade name Actilight from Beghin-Meiji Industries, Neuilly-sur-Seine, France. Inulin is also available under the trade name Frutafit from Sensus Operations C.V., Holland.

An especially preferred quality used in the invention is Raftilose Synergy 1 from Orafti which is an enriched chicory inulin containing a carefully selected DP (degree of polymerisation) distribution. It is a combination of chicory inulin molecules with selected chain lengths, enriched by a specific fraction of oligofructose produced by partial hydrolysis of chicory inulin.

Raftilose Synergy 1 thus contains a unique chain length distribution containing short, medium and long chain molecules. Since different chain lengths are known to be fermented at different rates, this leads to fermentation throughout the entire length of the colon. This is important in order to sustain a prebiotic effect in the whole of the colon.

The dosage size of inulin and oligofructose in the prebiotic combination product is in the range of 0.1 gram to 4 gram and more preferably in the range of 0.15 gram to 2 grams.

Resistant starch occurs naturally in a form physically inaccessible to α-amylase (RS₁), or as native starch that can be made accessible to the enzyme by gelatinisation (RS₂), or as retrograded starch formed through a cooking and cooling process (RS₃) and finally as a chemically modified starch (RS₄). RS₂, RS₃ and RS₄ are not digested by mammalian intestinal enzymes and are partly fermented in the colon and these three types are classified as Functional Fibre.

Resistant starch type RS₃ is obtained from Cerestar France S.A. under the trade name C*Actistar 11700. This is hydrolysed starch containing a high level of resistant starch (54 %), obtained from tapioca by enzymatic treatment. The resistant fraction is composed of retrograded amylase crystallites.

The dosage size of resistant starch dispensed as C*Actistar 11700 in the prebiotic combination product is in the range of 0.1 gram to 4 gram and more preferably in the range of 0.15 gram to 2 grams.

Resistant starch can also be supplied from National Starch and Chemical Co. under the trade name Novelose and Hi-maize. Novelose 330, Novelose 240 and Novelose 260 contain 30, 40 and 60% of resistant starch respectively. Hi-maize contains 60% of resistant starch. Novelose 330 and 240 contain resistant starch, type R₃ whereas Novelose 260 and Hi-maize both contain resistant starch, type R₂.

Suitable soluble fillers or diluents are non-cariogenic polyol qualities which are suitable for direct compression. Polyols like sorbitol, maltitol, mannitol, isomalt and xylitol are suitable as diluents. Examples of qualities suitable for direct compression are Xylitol directly compressible grade 300 from Danisco and Neosorb from Roquette Freres.

A diluent fibre component consisting of insoluble oat fibre is also used in the prebiotic combination product. This is supplied by J. Rettenmaier & Söhne GMBH, Germany under the trade names Vitacel Oat Fibre HF 600-30 and Vitacel Oat Fibre HF 101. Grade HF 600-30 is a microfine dietary fibre product produced by a special process from oats and especially suitable for inclusion into liquid products or powders to be dispensed into water whereas grade HF 101 is a cubic shaped particle and more suitable for inclusion into tablets. The insoluble diluent fibre is used in a concentration of up to 4 grams per unit dosage.

The two prebiotic components can be combined with a probiotic strain or a combination of probiotic strains to create a synbiotic product. The combination of the two prebiotic components and the probiotic strain(s) act in a synergetic fashion with respect to gut health and immune modulation. The two prebiotic components will ensure that the co-administered beneficial probiotic strain(s) will have a substrate to feed on in the lower small intestine and in the colon and thus to ensure an increased survival rate and multiplication in the gut ecosystem.
Suitable probiotic strains are selected from Bifidobacterium bifidum, B. infantis, B. lactis, B. longum, Lactobacillus acidophilus, L. brevis, L. bulgaricus, L. casei, L. plantarum, L. rhamnosus, L. salivarius and L. lactis. All the probiotic strains can be obtained from Rhodia Food. The probiotic strains from Rhodia Food also come in a special stabilized form called "RT-grade" which is especially suitable for tablet formulations. These stabilized forms of the probiotic strains consist of a protective membrane consisting of a saccharide/salt complex.

The two prebiotic components can be combined with suitable sources of calcium, magnesium, isoflavones, soy and vitamins.

Calcium carbonate is a preferred calcium source as this salt contains a high amount of calcium (40%), is well tolerated, shows a satisfactory bioavailability and is relatively inexpensive. When calcium carbonate is granulated it is important that the mean particle size is in the range of 5 to 25 µm. A particle size in this range will ensure a satisfactory chewability without the feeling of grittiness. Sturcal M from Speciality Minerals is suitable for this purpose.

A direct compressible quality of calcium carbonate is chosen in cases where the prebiotic components are made by direct compression. ForMaxx CaCO₃ 70 from Merck and Dicom^{™} L100 from Speciality Minerals are suitable qualities for this purpose. A direct compressible quality of calcium lactate (Puracal® DC from Purac) may also be used.

A calcium source is combined with the two prebiotic ingredients in an amount of 250 to 600 mg of elemental calcium per dosage form.

Magnesium lactate dihydrate is a preferred source of magnesium as this has got a high content of magnesium (10.3%), is bioavailable and is well tolerated. Magnesium lactate dihydrate from Moes with a mean particle size in the range of 50 to 120 µm is suitable for this purpose. Puramex® DC from Purac is a suitable quality of magnesium lactate which can be used for direct compression.
A magnesium source is combined with the two prebiotic ingredients in order to give between 50 and 120 mg of elemental magnesium per dosage form.

The prebiotic combination can be combined with suitable sources of soy powder and particular more refined soy ingredients containing a high level of isoflavones (daidzein, genistein and glycitein). Isoflavones are phytoestrogens which is an alternative to hormone replacement therapy (HRT) in the prevention of menopause related bone loss in women. Additionally supplementation with isoflavones offers menopausal benefits like a reduction of hot flushes and also contributes to a healthy heart. Combining the two prebiotic components with isoflavones, calcium, vitamin D₃ and vitamin K will result in an increased effect with respect to the prevention of bone loss in women. Suitable sources of isoflavones supplied from Cargill Health & Food Technologies are AdvantaSoy™ Complete Soy Isoflavones, AdvantaSoy™ Clear Soy Isoflavones and AdvantaSoy™ Compress Soy Isoflavones containing 2.3%, 45% and 50% of isoflavones respectively. Especially AdvantaSoy™ Compress Soy Isoflavones is suitable for direct compression and inclusion into chewable tablets.
Suitable sources are also Novasoy® 200 and Novasoy® 700 from Archer Daniels Midland Company (ADM) containing 20% and 70% of isoflavones respectively.
The content of isoflavones in the examples is in the range of 10 to 200 mg and more specifically between 25 and 100 mg.

Vitamin D₃ is included in the dosage forms containing a calcium source together with the two prebiotic components. A sufficient supply of vitamin D₃ is important in order to ensure a satisfactory absorption of calcium from the intestine.
Vitamin D₃ 100CWS from Roche is suitable for this purpose as it is cold water soluble and with a long shelf life. Vitamin D₃ is included in the dosage forms in a range of 2.5 µg (100IU) cholecalciferol to 20 µg (800IU) cholecalciferol per dosage form.

Vitamin K₁ can be included in the dosage forms containing a calcium source as it has been found that low intake levels of vitamin K in the elderly population has been associated with low bone mineral density and bone fractures. Vitamin K₁ is available from Roche as Dry Vitamin K₁ 5% SD, a dry substance containing 5% vitamin K₁. Vitamin K₁ is included in order to give 0.05 to 5 mg vitamin K₁ per dosage form.

Vitamins may also be combined with the two prebiotic ingredients to constitute instant powders to be dissolved in liquids. Suitable qualities are cold water soluble and can be supplied from Roche or BASF.

Taste masked vitamin qualities are used where the prebiotic combination is used together with direct compressible vitamins in chewable tablets. The Rocoat range of vitamins supplied from Roche is suitable for this purpose.

The prebiotic combinations of inulin, oligofructose and resistant starch optionally together with a source of calcium, magnesium, vitamin D₃, vitamin K₁, isoflavones and probiotic strain(s) are especially suitable for the formulation of chewable tablets due to the small dosage size per unit of the two prebiotic components. Chewable tablets are a preferred formulation as this is easy to take for the patient or consumer and thus increase the compliance of the product.
Chewable tablets can be produced either by direct compression or by wet granulation and subsequent drying of the prebiotic components in a fluid bed.

### Method I:

Enriched inulin as Synergy 1 may be used for direct compression, but also especially compression grades of inulin made for direct compression can be chosen. Examples of these are Raftiline GR. from Orafti and Frutafit IQ from Sensus. The free-flowing granular inulin quality is then further blended with resistant starch RS₃ or RS₂ component, a suitable diluent, acidulent, flavour, optionally a sweetener and tablet lubricant to constitute a chewable tablet. Suitable diluents are non-cariogenic polyol qualities which are suitable for direct compression. Polyols like sorbitol, maltitol, mannitol, isomalt and xylitol are suitable as diluents. Examples of qualities suitable for direct compression are Xylitol directly compressible grade 300 from Danisco and Neosorb from Roquette Freres. Acidulants are added in order to complement the flavour and these are commonly chosen from malic and citric acid. Suitable flavours which are free-flowing and with a long shelf-life are of the Durarome flavour range from Firmenich. Sweeteners like aspartame and acesulfam K are suitable for inclusion into the chewable tablet. Tablet lubricants like magnesium stearate, sodium stearyl fumarate and polyethylene glycol 6000 or 8000 may be used where magnesium stearate is the preferred quality.
The tablet end mixture is compressed into chewable tablets with a tablet weight in the range of 700 to 2800 mg and preferably in the range of 1000 mg to 2100 mg.

A granulation method may be employed for the chewable formulations as this will improve flow and compression characteristics in high speed tableting machines as described below:

### Method II:

The two prebiotic components together with the polyol diluent is granulated in a fluid bed granulation and drying equipment. Examples of fluid bed equipments are pilot models like GPCG 3 from Glatt and Multiprosessor from Niro Aeromatic. The granulation takes place by top spray with a spray atomisation pressure of 1.5 bars and a liquid spray rate of 20 to 50 g/min depending on the particular binder and concentration used. Granulation and drying takes place with an inlet air temperature of the fluidisation air of 40 to 80°C and with a batch size of 2.0 - 3.5 kg. The granulation liquid is used in a concentration of 10 to 40% depending on the particular binder used. Oligofructose which is a prebiotic can be used as a binder in a concentration of 40% and Raftilose P95 from Orafti is suitable for this purpose. Likewise inulin (Raftilose Synergy 1) can be used as a binder in a concentration of 15%. A combination of inulin and resistant starch (Actistar 11700) can also be used with a total content of dry matter in the range of 10 - 40%. Other commonly used binders can be used like polyvinylpyrrolidone (PVP) grade 30 or 90 and PVP VA64 and be used in a concentration range of 10 to 30%. Other commonly used binders like maltodextrin and various starch derivatives may also be used.
The fluidisation volume is adjusted during the granulation and drying process and is typically in the range of 20 to 120 m³/h. The total processing time is in the range of 15 to 30 min depending upon the spray rate and inlet air temperature employed.
The granulation and drying in the fluid bed apparatus results in a free-flowing granulate with a residual water content of 1 to 4%. The granulate has typically a mean particle size of 80 to 250 µm which makes it free-flowing and suitable for mixing with other ingredients. The granulate is further blended with an acidulant, flavour component, optionally an artificial sweetener and a tablet lubricant

### Method III:

The granulation may alternatively also be carried out in a high speed mixer. A suitable pilot model is a vertical high speed mixer like Fielder PMA 25/2G. The granulation takes place with a granulation liquid according to method II. The batch size is 4 - 5 kg and the dry ingredients are first mixed for 1 min with the main impeller and knife at the highest speed. The granulation liquid is atomised with a two-fluid nozzle and with a spray pressure of 2 bar and with a spray rate of 60 g/min. 300 gram of a granulation liquid is sprayed on to the moving powder bed with the main impeller at the highest speed (400 RPM). The wet granulate is then mixed for another 1 min before being transferred to a fluid bed for drying with an inlet temperature in the fluidising air of 70 to 80°C. The resultant granulate has typically a mean particle size in the range of 100 to 300 µm.

The described prebiotic combination products are to be used for the following conditions and diseases.

Inflammatory bowel disease: The prebiotic combination products are taken in order to avoid the symptoms and acute attacks connected to ulcerative colitis, Crohn's disease and irritable bowel syndrome.

Enhanced mineral bioavailability: The prebiotic combination of enriched inulin and resistant starch in combination with calcium and vitamin D₃ will both increase the absorption of calcium and at the same time exert a beneficial bifidogenic effect in the colon. This combination will especially be beneficial for post-menopausal women who are at risk of developing osteoporosis and who at the same time also suffer from digestive disorders like constipation.

Food intolerance: Intake of the prebiotic combination of enriched inulin and resistant starch will cause a favourable colonic ecosystem and fermentation pattern which will allow the host to digest foodstuffs which otherwise would cause digestive problems.

Digestive disorders: Inulin, oligofructose and resistant starch have a mild laxative effect. The mechanism works via stimulation of the microbial growth with a concomitant increase in bacterial mass, and thus stimulation of peristalsis by the increased bowel content.

Immune modulation: Allergy, manifested in atopic eczema, allergic rhinoconjunctivitis and asthma, represent an epidemic of rising importance. T helper cells as Th1 and Th2 play a central role in the adaptive immune responses. The immunological basis of allergy orginates from an over expression of type 2 T helper cells whereas autoimmune diseases orginates from an overexpression of type 1 T helper cells.
It is thought that prebiotics and probiotics help to modulate the balance between Th1 and Th2 in such a way as to prevent the development of allergic and autoimmune diseases.

Traveller's diarrhoea: A prophylactic intake of the prebiotic combination product before a travel and during the stay at a foreign destination will ensure that the host has a robust enteric microflora which can withstand pathogenic microbes in contaminated food and drink.
A special case is the need for athletes to stay well before and during important sport events where they participate. Intake of the prebiotic combination product will make it less likely that they fall ill with a gastrointestinal infection and also support their uptake of important minerals like calcium and magnesium.

Restoration of the enteric microflora after a cure with antibiotics: A cure of antibiotics will often eradicate the existing microflora and the restoration of the microflora may cause a lot of gastrointestinal problems. This can be greatly reduced with the prebiotic combination product which will aid the colonisation by beneficial bifidus and lactobacillus microorganisms.

Prophylaxis of colon cancer: The prebiotic combination produces butyrate in the colon which is an important regulator of colonic cell growth and differentiation. It has been observed that the incidence of colon cancer is inversely related to the intake of starch, in particular resistant starch and that diets high in resistant starch appear to provide protection against colon cancer.

Energy management: Several studies have measured the glycaemic response to foods containing high-amylose maize starch, in healthy non-diabetic, non-insulin-dependent diabetes mellitus and hyperinsulinemic subjects. These studies show a consistent trend whereby high-amylose maize starch in foods can impact upon the glycaemic response, including lower maximum blood-glucose response, reduced area under the blood-glucose response curve and under the blood-insulin response curve.

### Examples

### Example 1

A prebiotic free flowing powder consisting of three types of dietary fibres with the following composition per 5 ml measuring spoon.

| | |
|---|---|
| Inulin and oligofructose (Raftilose Synergy 1): | 0.7 gram |
| Resistant starch (Actistar 11700): | 0.7 gram |
| Oat fibre fine size (Vitacel oat fibre 600-30) | 0.7 gram |

| | |
|---|---|
| Dispersion in a glass of tap-water: | 20 seconds |
| Bulk density: | 0.44 g/ml |
| Taste: | Neutral or bland |

The ingredients are mixed in a high speed mixer like a Kenwood Major for 2 min on speed 5 or a Fielder PMA 25/2G with for 2 min with main impeller on lowest speed.

The prebiotic free flowing powder is packed in a 500 ml container with a 5 ml measuring spoon where one dosage of 5 ml is equivalent to 2.1 grams. The prebiotic powder blend can alternatively be packed into sachets each holding 2.1 grams.

### Example 2

A consumer acceptance study was performed with the product in example 1. Eighteen subjects participated in the study which took place over a period of five weeks. The number bowel movements to pass faeces and the degree of bloatedness, intestinal pressure, intestinal sounds and the consistency of the faeces were recorded after each week. The results were recorded after each week on a visual-analogue scale where 0: no discomfort, 2.0: a little discomfort, 4.0: some discomfort, 6.0: a lot of discomfort and 8.0: an unacceptable degree of discomfort. The number of faecal passages was noted each day.

The first week consisted of a control period where the prebiotic supplement was not taken. During the next week one dosage of 2.1 g was taken in the morning. During the last three weeks two dosages were taken each day; one in the morning and one at night. Among the eighteen subjects there were two subjects with ulcerative colitis and two subjects with food intolerances. At the end of the acceptance study these four subjects reported that the prebiotic supplement had caused an improvement with respect to the gastrointestinal problems related to their illnesses.

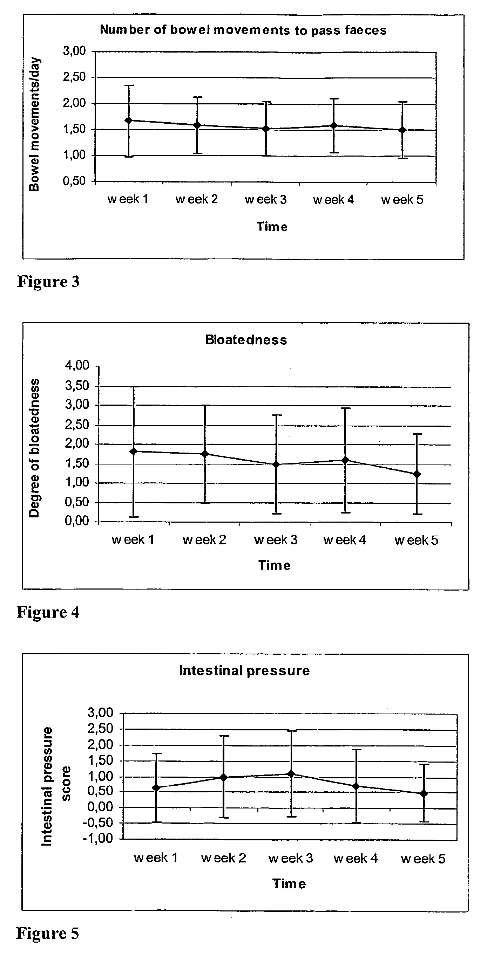

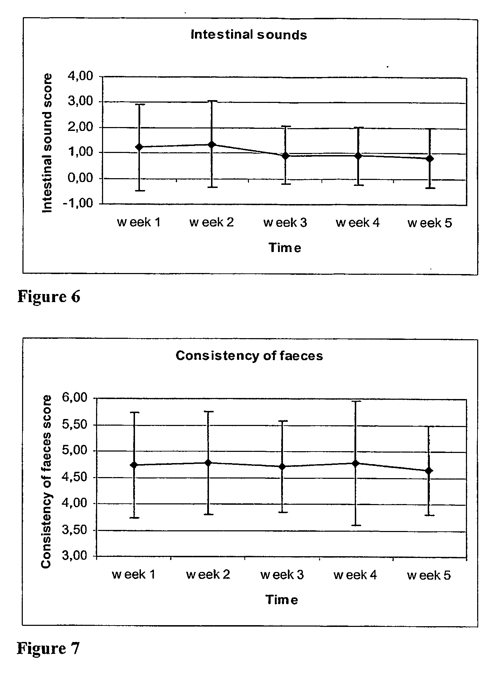

The results from the acceptance study over 5 weeks are depicted in figure 3, 4, 5, 6 and 7.

The results after the second, third, fourth and fifth week were corrected by subtracting the result from week 1. The differences were compared to 0 by a two-tailed T-test. There were no statistical differences in any of the measured parameters when the results from the test periods were compared to the control week.

The results show that the dietary supplement is very well tolerated when the supplement is taken once or twice daily.

The two subjects with ulcerative colitis and one subject with food intolerance participated in a new long term study over six months which had the aim of finding the minimum effective dosage and to monitor long term effect of the dietary supplement with respect to efficacy and tolerance. The three subjects had the following diagnoses:
Subject 1: 50 year old male with diagnosis of ulcerative colitis since 1993 and with prescribed medication of 2x2 of 500mg sulfasalazin (Salazopyrin) daily.
Subject 2: 29 year old female with diagnosis of ulcerative colitis since 1991 with prescribed medication of 2x2 of 500 mg sulfasalazin daily and additionally with courses of steroid treatment (2x5mg prednisolon x 2) in connection to acute attacks of ulcerative colitis.
Subject 3: 25 year old female with 7 years of food intolerance in connection to the intake of several foods with resulting bloatedness , stomach pains and diarrhoea. In addition suffering from gastrointestinal candida infections.

The two subjects suffering from ulcerative colitis had continued taking the prebiotic supplement after the acceptance study due to a beneficial effect with respect to the degree of the symptoms of ulcerative colitis, e.g. they felt that the prebiotic prevented incidences or outbreaks of the disease. At the start of the long term study they could report that they did not take any medication other than the prebiotic supplement. The subject with the food intolerance had not taken the prebiotic supplement between the two studies.

The study took part over a period of 6 months with registrations of results after week 1 and then after the first week in month 2, 4 and 6. The test person started by taking one dosage (5 ml) daily of the prebiotic supplement in week 2 and then was told to adjust the dosage in order to find the minimum effective dosage that gave a significant relief of the main symptom(s) connected to their illness.
The subjects were asked to register the number of passed faeces per day, the main symptom(s) of their illness and the degree of relief of this symptom, any gastrointestinal symptom related to the prebiotic supplement and the consistency of the faeces. The test persons registered the results by marking the score on a visual analogue scale as shown below.

| No discomfort | A little discomfort | Some discomfort | A lot of discomfort | Unacceptable discomfort |
|---|---|---|---|---|
| 0 | 2,5 | 5 | 7,5 | 10 |

They were asked to comment on the effectiveness of the prebiotic supplement after each registration period.

| | | Subject 1 | Subject 2 | Subject 3 |
|---|---|---|---|---|
| Main symptom of the illness | | Watery faeces with occasional bleeding | Watery faeces with occasional bleeding, pains in the left side of the abdomen | Stomach pains, diarrhoea, bloatedness |
| Degree of severity of symptom | week 1 | 0.9 | 3.5 | 5.0 |
| | week1/2 month | 0.7 | 5 | 5.0 |
| | week1/4 month | 0.3 | 3.0 | 2.5 |
| | week1/6 month | 0.2 | 0.8 | 2.5 |
| Average number of passed faeces per day | week 1 | 1.3 | 1.5 | 1.9 |
| | week 1/2 month | 1.1 | 0.9 | 1.7 |
| | week 1/4 month | 1.4 | 0.9 | 1.7 |
| | week 1/6 month | 1.3 | 0.9 | 1.7 |
| Consistency of faeces | week 1 | 5.8 | 5.8 | 7.5 |
| | week 1/2 month | 5.5 | 2.4 | 6.2 |
| | week 1/4 month | 5.5 | 3.0 | 6.2 |
| | week 1/6 month | 5.8 | 6.2 | 6.2 |
| Dosage per day | week 1 | 1 | 1 | 1 |
| | week 1/2 month | 1 every other day | 1 | ½ every other day |
| | week 1/4 month | 1 every third day | 1 every other day | ½ every other day |
| | week 1/6 month | 1 every third day | 1 every third day | ½ every other day |

Throughout the study the two persons with ulcerative colitis reported a good control with respect to the symptoms of the disease and a normal defecation pattern of one to two passed faeces per day. There were no episodes of outbreaks of ulcerative colitis with slimy and watery diarrhoea together with bleeding. At the end of the study both subjects reported a minimum effective dosage of one measuring spoon (5ml containing 2.1 gram) taken every third day.
Likewise the subject with food intolerance reported an improvement in the main symptoms of the illness. At the end of the study the subject was able to tolerate the intake of more foodstuffs without experiencing any sign of stomach pains or bloatedness. The minimum effective dosage for this subject was ½ measuring spoon every other day.

Thus it has been found a surprisingly low minimum effective dosage for the prebiotic fibre supplement in the prophylactic treatment of ulcerative colitis and food intolerance. The minimum effective dosage for the enriched inulin (Raftilose Synergy 1) and resistant starch (Actistar 11700) components are in the range of 175 to 233 mg per day for both the components.
The discovery of the very low minimum effective dosages for the two prebiotic components suggests that there is a synergistic effect between the two components. The very low dosages of the two prebiotics makes it possible to make new and cost-effective dosage forms with a good compliance. The good compliance is both a result of the compact dosage form with good sensoric properties but also because of the lack of side-effects like flatulence and bloatedness.

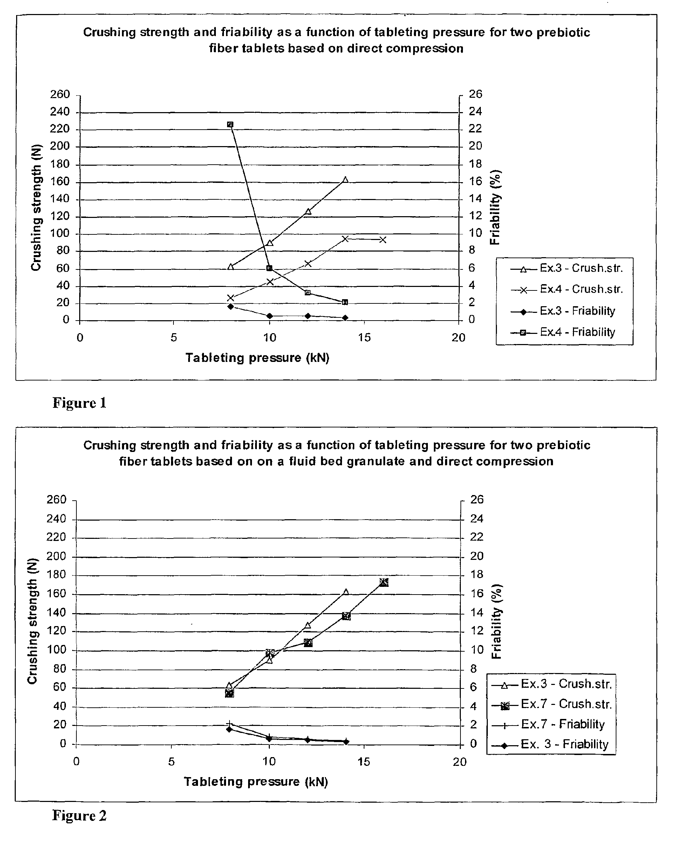

The tablet characteristics, sensoric properties and resistance towards moisture for the chewable formulations described in the invention was investigated by comparing a formulation consisting of enriched inulin in combination with resistant starch with a formulation consisting of enriched inulin alone. The tablet characteristics of the two formulations were compared by plotting the tableting pressure against the crushing strength and friability of the two tablet formulations (figure 1). Example 3 consists of 722 mg inulin/oligofructose as Raftilose Synergy 1 and 703 mg resistant starch as Actistar 11700 constituting as much as 93% of fiber per tablet. Example 4 consists of 92% of Raftilose Synergy 1 only.
The two formulations were exposed to tableting pressures of 8, 10, 12,14 and 16 kN and the crushing strength and friability of the collected samples were measured. The compression curve for example 3 showed a linear curve up to very high tableting pressures without any tendency of capping or scale-off whereas example 4 showed levelling off at higher tableting pressure with the production of faulty tablets. Chewable tablets need to have a sufficient low friability below 2% in order to withstand bulk handling and packaging. A normal crushing strength for chewable tablets is in the range of 50 to 80 Newton. As can be seen from figure 1 the friability values for example 3 are all below 2% whereas the friability values for example 4 never come below 2% even at high tableting pressures. Tablets based on the oligofructose or inulin alone will thus not have the required low value for the friability and the formulation will not be suitable for a tablet production on an industrial scale.

Figure 2 show tableting pressure against crushing strength and friability for two formulations both containing approximately 92-93% of prebiotic fiber but where one formulation (ex.3) is based on direct compression and the other formulation is based on fluid bed agglomeration (ex.7). Both the curves for crushing strength and friability follow each other closely which proves that the beneficial effect of resistant starch is not a result of a granulation method but a result of the formulation itself.
The two formulations (example 3 and 4) were compared with respect to sensoric properties. Example 3 based on the combination between inulin/oligofructose and resistant starch showed superior properties with respect to dispersion in the mouth and hardly any toothpacking could be perceived. Example 4 on the other hand did not disperse well in the mouth and a high degree of toothpacking could be detected with the formation of a chewy lump in the mouth.
The two formulations were also compared with respect to moisture uptake where it was shown that the example 3 was much more resistant towards moisture than the formulation based on example 4.

The following examples visualise the new dosage forms which are made possible due to the new discovery.

Example 3, 4, 5 and 6: These four examples are formulated as chewable tablets based on direct compression. The tablets were made on a Manesty B3B rotary tableting machine with a tableting speed of 45 000 tablets per min and with a crushing strength or hardness of the tablets which was in the range of 50 to 80 N.

| | Ingredient | Ex. 3 | Ex. 4 | Ex. 5 | Ex. 6 |
|---|---|---|---|---|---|
| 1 | Inulin and oligofructose (Raftilose | 722 mg | 1426 mg | 350 mg | 200 mg |
| | Synergy 1) | | | | |
| 2 | Resistant starch (Actistar 11700) | 703 mg | | 350 mg | 200 mg |
| 3 | Xylitol (fine granular grade 300) | 95 mg | 88 mg | 175 mg | 100 mg |
| 4 | Dicom L100 | | | | 1250 mg |
| 5 | Calcium carbonate (Formaxx CaCO₃ 70) | | | 893 mg | |
| 6 | AdvantaSoy™ Compress | | | | |
| 7 | Vitamin D₃ (100 CWS) | | | 2.2 mg | 2.2 mg |
| 8 | Durarome lemon flavour | | | 15 mg | |
| 9 | Durarome strawberry flavour | 15 mg | 14 mg | | 15 mg |
| 10 | Malic acid | 10 mg | 9 mg | | 10 mg |
| 11 | Aspartame | | | 2 mg | 2 mg |
| 12 | Magnesium stearate | 8 mg | 7.0 mg | 7 mg | 8 mg |
| | Tablet weight | 1553 mg | 1544 mg | 1794 mg | 1788 mg |
| | Tablet diameter | 16 mm | 16 mm | 16 mm | 16 mm |

All the ingredients were blended according to example 1 and 16 mm normal convex shaped chewable tablets made.

### Example 7-10:

A range of chewable tablets based on granulation in a fluid bed or high speed mixer (method II or III) have been formulated. Ingredients no. 1 to 7 are all granulated in the respective examples. The resultant granulate is further mixed with ingredients 8 to 16 which are added in the dry state and mixed according to example 1.

Several compositions of granulation liquid were employed and were as follows:

| | |
|---|---|
| Granulation liquid type A: | 15% Synergy 1 and 10% Actistar 11700 |
| Granulation liquid type B: | 15% Synergy 1 |
| Granulation liquid type B: | 25% PVP VA64 |

Chewable tablets were made according to example 3 - 6 and with the following compositions:

| | Ingredient | Ex. 7 | Ex. 8 | Ex. 9 | Ex. 10 |
|---|---|---|---|---|---|
| 1 | Inulin and oligofructose (Raftilose Synergy 1) | 713 mg | 175 mg | 280 mg | 320 mg |
| 2 | Resistant starch (Actistar 11700) | 694 mg | 200 mg | 273 mg | 320 mg |
| 3 | Oat fibre fine size (Vitacel oat fibre 600-30) | | | 261 mg | |
| 4 | Xylitol CM 50 | 95 mg | | 151 mg | 200 mg |
| 5 | Calcium carbonate (Sturcal M) | | 1250 mg | | |
| 6 | Magnesium lactate dihydrate (Moes) | | | | 781 mg |
| 7 | PVP VA64 | | 28 mg | | |
| 8 | Vitamin D₃ (100 CWS) | | 2.2 mg | | |
| 9 | Vitamin K₁ 5% SD | | | | |
| 10 | Durarome lemon flavour | | 12 mg | | |
| 11 | Durarome peach flavour | | | | 10 mg |
| 12 | Durarome strawberry flavour | 15 mg | | | |
| 13 | Durarome raspberry flavour | | | 15 mg | |
| 14 | Malic acid | 10 mg | 10 mg | 10 mg | 10 mg |
| 15 | Aspartame | | | 2 mg | 1 mg |
| 16 | Magnesium stearate | 8 mg | 8 mg | 8 mg | 8 mg |
| | Tablet weight | 1535 mg | 1685 mg | 1000 mg | 1650 mg |
| | Tablet diameter | 16 mm | 16 mm | 16 mm | 16 mm |
| | | | | | |
| | Granulation batch size | 2.4 kg | 3.0 kg | 2.0 kg | 3.0 kg |
| | Granulation liquid type | A | C | | B |
| | ranulation liquid amount | 400 g | 300 g | 300 g | 300 g |
| | Granulation method | II | II | II | II |

| | Ingredient | Ex. 11 | Ex. 12 | Ex. 13 | Ex. 14 |
|---|---|---|---|---|---|
| 1 | Inulin and oligofructose (Raftilose Synergy 1) | 211 mg | 500 mg | 230 mg | 500 mg |
| 2 | Resistant starch (Actistar 11700) | 211 mg | 900 mg | 270 mg | 500 mg |
| 3 | Oat fibre fine size (Vitacel oat fibre HF 101) | | | | 500 mg |
| 4 | Xylitol CM 50 | | 100 mg | 120 mg | 100 mg |
| 5 | Calcium carbonate (Sturcal M) | 1250 mg | | 1000 mg | |
| 6 | Magnesium lactate dihydrate (Moes) | | | | |
| 7 | Vitamin D₃ (100 CWS) | 2.2 mg | | 4.4 mg | |
| 8 | AdvantaSoy™ Compress | | | 50 mg | |
| 9 | Vitamin K₁ 5% SD | 10 mg | | 10 mg | |
| 10 | Durarome lemon flavour | | 15 mg | 12 mg | |
| 11 | Durarome peach flavour | 10 mg | | | 10 mg |
| 12 | Durarome banana flavour | 5 mg | | | 5 mg |
| 13 | Malic acid | 10 mg | 10 mg | | 8 mg |
| 14 | Aspartame | 2 mg | 1 mg | 1 mg | 1 mg |
| 15 | Magnesium stearate | 9 mg | 8 mg | 8 mg | 9 mg |
| | Tablet weight | 1720 mg | 1534 mg | 1705 mg | 1633 mg |
| | Tablet diameter | 16 mm | 16 mm | 16 mm | 16 mm |
| | | | | | |
| | Granulation batch size | 3.0 kg | 2.4 kg | 3.0 kg | 5.0 kg |
| | Granulation liquid | B | A | B | B |
| | Granulation amount | 400 g | 300 g | 400 mg | 300 g |
| | Granulation method | II | II | II | III |

### Example 15:

A tablet to be swallowed is formulated in the below example. A prebiotic granulate is first made according to example 7:

| | Per tablet |
|---|---|
| Prebiotic granulate: | 730 mg |
| Inulin and oligofructose | |
| (Raftilose Synergy 1): | 346 mg |
| Resistant starch (Actistar 11700): | 337 mg |
| Xylitol CM 50 | 46 mg |
| AdvantaSoy™ Compress | 100 mg |
| Ac-di-sol (FMC) | 8 mg |
| Magnesium stearate | 4 mg |
| Sum tablet weight | 842 mg |

The tableting blend is compressed to give 12 mm normal convex shaped tablets to be swallowed.

The tablets may optionally be filmcoated where the film coating contain a low viscosity grade of hydroxypropylmethylcellulose (film former) , polyethylene glycol 400 (plasticizer), titanium dioxide (occluding pigment), iron oxide pigment gelb (colour) and bees wax as polishing aid. -

### Example 16:

A synbiotic chewable tablet is formulated as shown below. A prebiotic granulate is first made according to example 7:

| | Per tablet |
|---|---|
| Prebiotic granulate: | 850 mg |
| Inulin and oligofructose | |
| (Raftilose Synergy 1): | 403 mg |
| Resistant starch (Actistar 11700): | 393 mg |
| Xylitol CM 50 | 54 mg |
| Bifidobacterium infantis (5 x 10¹⁰ CFU/g) grade RT | 100 mg |
| Bifidobacterium longum (5 x 10¹⁰ CFU/g) grade RT | 100 mg |
| Lactobacillus acidophilus La-14 (5 x 10¹⁰ CFU/g) grade RT | 100 mg |
| Durarome lemon flavour | 8 mg |
| Durarome peach flavour | 5 mg |
| Magnesium stearate | 7 mg |
| Sum tablet weight | 1170 mg |

The tableting blend is compressed to give 14 mm normal convex shaped chewable tablets.

### Example 17:

A prebiotic and multivitamin chewable tablet is formulated as shown below. A prebiotic granulate is first made according to example 7:

| | | Label claim | Overage | Per |
|---|---|---|---|---|
| tablet | | | | |
| Prebiotic granulate: | | | | 850mg |
| Inulin and oligofructose | | | | |
| (Raftilose Synergy 1): | 403 mg | 400 mg | | |
| Resistant starch (Actistar 11700): | 393 mg | 400 mg | | |
| Xylitol CM 50 | 54 mg | | | |
| Dry Vitamin A Acetate 500 | | 400 µg | 20% | 2.90 mg |
| Dry Vitamin D₃ 100 CWS | | 5.0 µg | 10% | 2.2 mg |
| Dry Vitamin E Acetate 950 NS | | 30 TE | 10% | 51.80 mg |
| Ascorbic acid 90% granulation | | 100 mg | 10% | 122.20 mg |
| Sodium ascorbate | | 100 mg | 10% | 123.60 mg |
| Rocoat Thiamine Mononitrate 33.3% | | 1.2 mg | 20% | 4.50 mg |
| Rocoat Riboflavine 33.3% | | 1.3 mg | 10% | 4.30 mg |
| Rocoat Pyridoxine Hydrochloride 33.3% | | 1.2 mg | 10% | 4.80 mg |
| Vitamine B₁₂ 0.1% WS | | 2.0 µg | 10% | 2.20 mg |
| Rocoat Niacinamide 33.3% | | 15.0 mg | 10% | 49.90 mg |
| Folic acid 10% Fructose Trituration | | 200 µg | 20% | 2.0 mg |
| Xylitol (fine granular grade 300) | | | | 192.60 mg |
| Durarome lemon flavour | | | | 20.00 mg |
| Durarome peach flavour | | | | 10.00 mg |
| Magnesium stearate | | | | 7.00 mg |
| Sum tablet weight | | | | 1450 mg |

The tableting blend is compressed to give 16 mm normal convex shaped chewable tablets.

The prebiotic combination may also be included into other dosage forms and foods like effervescent tablets, emulsions, liquid mixtures, soft drinks, yoghurts and juice concentrates.

## Claims

1. A chewable tablet comprising a prebiotic combination product of inulin and/or oligofructose and resistant starch, and wherein said combination product has been subjected to direct mixing and compression or a wet granulation process and drying before tableting

2. A chewable tablet according to claim 1, wherein the amount of inulin and/or oligofructose and resistant starch is in the range of 0.1 g to 4g.

3. A chewable tablet according to claim lor claim 2, wherein the amount of inulin and/or oligofructose and resistant starch is in the range of 0.15g to 2g.

4. A chewable tablet according to any of the claims 1 to 3, wherein the tablet further comprises probiotics.

5. A chewable tablet according to any of the claims 1 to 4, wherein said inulin and/or oligofructose is produced from the chicory plant or short chain fructooligosaccharides derived from sugar cane by an enzymatic process.

6. A chewable tablet according to any of the claims 1 to 5, wherein said resistant starch is produced from the tapioca plant or the maize plant.

7. A chewable tablet according to any of the claims 1 to 6, wherein said tablet further comprises soy and/or isoflavones from soy or other sources.

8. A chewable tablet according to any of the claims 1 to 7, wherein said tablet further comprises any of the components selected from the group comprising calcium, magnesium or vitamins.

9. A chewable tablet according to any of the claims 1 to 8, wherein said tablet further comprises oat fibre.

10. A chewable tablet product according to any of the claims 1 to 9, wherein said product can be in the form of a dietary supplement or a medicament.

11. A chewable tablet according to any of the claims 1 to 10 used as a medicament.

12. Use of a prebiotic combination comprising inulin and /or oligofructose and resistant starch for the preparartion of a medicament in form of a chewable tablet for prophylaxis or treatment of inflammatory bowel disease.

13. Use of a prebiotic combination comprising inulin and /or oligofructose and resistant starch for the preparartion of a medicament in form of a chewable tablet for prophylaxis or treatment of food intolerance.

14. Use of a prebiotic combination comprising inulin and /or oligofructose and resistant starch for the preparartion of a medicament in form of a chewable tablet for prophylaxis or treatment of, calcium and magnesium deficiency including osteoporosis, the general regulation of the digestion, immune modulation, energy management, diarrhoea, prophylaxis of colon cancer and protection of the gut microbial ecosystem in man, and restoration of the enteric microflora after a cure with antibiotics.

## Patentansprüche

1. Kaubare Tablette umfassend ein präbiotisches Kombinationsprodukt aus Inulin und/ oder Oligofruktose und resistenter Stärke, und wobei das Kombinationsprodukt direktem Mischen und einer Kompression oder einem Feuchtgranulierungsverfahren und Trocknen vor dem Tablettieren unterzogen wurde.

2. Kaubare Tablette nach Anspruch 1, wobei die Menge an Inulin und/oder Oligofruktose und resistenter Stärke in dem Bereich von 0,1 g bis 4 g ist.

3. Kaubare Tablette nach Anspruch 1 oder Anspruch 2, wobei die Menge an Inulin und/oder Oligofruktose und resistenter Stärke in dem Bereich von 0,15 g bis 2 g ist.

4. Kaubare Tablette nach einem der Ansprüche 1 bis 3, wobei die Tablette ferner Probiotika umfasst.

5. Kaubare Tablette nach einem der Ansprüche 1 bis 4, wobei das Inulin und/oder die Oligofruktose erzeugt wird aus der Chicoreepflanze oder kurzkettigen Fruktooligosacchariden, abgeleitet aus Zuckerrohr durch ein enzymatisches Verfahren.

6. Kaubare Tablette nach einem der Ansprüche 1 bis 5, wobei die resistente Stärke hergestellt worden ist aus der Maniokpflanze oder der Maispflanze.

7. Kaubare Tablette nach einem der Ansprüche 1 bis 6, wobei die Tablette ferner umfasst Soja und/oder Isoflavone aus Soja oder anderen Quellen.

8. Kaubare Tablette nach einem der Ansprüche 1 bis 7, wobei die Tablette ferner umfasst beliebige Komponenten ausgewählt aus der Gruppe umfassend Kalzium, Magnesium oder Vitamine.

9. Kaubare Tablette nach einem der Ansprüche 1 bis 8, wobei die Tablette ferner Haferfaser umfasst.

10. Kaubares Tablettenprodukt nach einem der Ansprüche 1 bis 9, wobei das Produkt in Form eines Nahrungsergänzungsmittels oder eines Medikaments sein kann.

11. Kaubare Tablette nach einem der Ansprüche 1 bis 10, welche als Medikament verwendet wird.

12. Verwendung einer präbiotischen Kombination umfassend Inulin und/oder Oligofruktose und resistente Stärke zur Herstellung eines Medikaments in Form einer kaubaren Tablette zur Prophylaxe oder Behandlung von entzündlicher Darmerkrankung.

13. Verwendung einer präbiotischen Kombination umfassend Inulin und/oder Oligofruktose und resistente Stärke zur Herstellung eines Medikaments in Form einer kaubaren Tablette zur Prophylaxe oder Behandlung von Nahrungsmittelintoleranz.

14. Verwendung einer präbiotischen Kombination umfassend Inulin und/oder Oligofruktose und resistente Stärke zur Herstellung eines Medikaments in Form einer kaubaren Tablette zur Prophylaxe oder Behandlung von Kalzium- und Magnesiumdefizienz, umfassend Osteoporose, der allgemeinen Regulierung der Verdauung, Immunmodulation, Energieverwaltung, Diarrhö, zur Prophylaxe von Kolonkrebs und zum Schutz des mikrobiellen Ökosystems des Darms im Menschen und zur Wiederherstellung der enterischen Mikroflora nach einer Behandlung mit Antibiotika.

## Revendications

1. Comprimé à mâcher comprenant un produit de combinaison prébiotique composé d'inuline et/ou d'oligofructose et d'amidon résistant, et dans lequel ledit produit de combinaison a été soumis à un mélange direct et à une compression ou à un procédé de granulation par voie humide et à un séchage avant la fabrication des comprimés.

2. Comprimé à mâcher selon la revendication 1, dans lequel la quantité d'inuline et/ou d'oligofructose et d'amidon résistant se situe dans la plage de 0,1 g à 4 g.

3. Comprimé à mâcher selon la revendication 1 ou la revendication 2, dans lequel la quantité d'inuline et/ou d'oligofructose et d'amidon résistant se situe dans la plage de 0,15 g à 2 g.

4. Comprimé à mâcher selon l'une quelconque des revendications 1 à 3, dans lequel le comprimé comprend en outre des probiotiques.

5. Comprimé à mâcher selon l'une quelconque des revendications 1 à 4, dans lequel ladite inuline et/ou ledit oligofructose est produit(e) (sont produits) à partir de la plante de chicorée ou de fructooligosaccharides à chaîne courte dérivant de la canne à sucre par un procédé enzymatique.

6. Comprimé à mâcher selon l'une quelconque des revendications 1 à 5, dans lequel ledit amidon résistant est produit à partir de la plante de tapioca ou de la plante de maïs.

7. Comprimé à mâcher selon l'une quelconque des revendications 1 à 6, dans lequel ledit comprimé comprend en outre du soja et/ou des isoflavones provenant du soja ou d'une autre source.

8. Comprimé à mâcher selon l'une quelconque des revendications 1 à 7, dans lequel ledit comprimé comprend en outre l'un quelconque des composants choisis dans le groupe comprenant le calcium, le magnésium ou les vitamines.

9. Comprimé à mâcher selon l'une quelconque des revendications 1 à 8, dans lequel ledit comprimé comprend en outre des fibres d'avoine.

10. Comprimé à mâcher selon l'une quelconque des revendications 1 à 9, dans lequel ledit produit peut être sous la forme d'un supplément diététique ou d'un médicament.

11. Comprimé à mâcher selon l'une quelconque des revendications 1 à 10, utilisé en tant que médicament.

12. Utilisation d'une combinaison prébiotique comprenant de l'inuline et/ou un oligofructose et un amidon résistant pour la préparation d'un médicament sous la forme d'un comprimé à mâcher pour la prophylaxie ou le traitement de la maladie intestinale inflammatoire.

13. Utilisation d'une combinaison prébiotique comprenant de l'inuline et/ou un oligofructose et un amidon résistant pour la préparation d'un médicament sous la forme d'un comprimé à mâcher pour la prophylaxie ou le traitement des intolérances alimentaires.

14. Utilisation d'une combinaison prébiotique comprenant de l'inuline et/ou un oligofructose et un amidon résistant pour la préparation d'un médicament sous la forme d'un comprimé à mâcher pour la prophylaxie ou le traitement de la carence calcique et magnésique comprenant l'ostéoporose, la régulation générale de la digestion, la modulation de l'immunité, la gestion de l'énergie, les diarrhées, la prophylaxie du cancer du côlon et la protection de l'écosystème microbien intestinal chez l'homme, et la restauration de la microflore entérique après un traitement avec des antibiotiques.
